(19) Europäisches Patentamt
European Patent Office
Office européen des brevets

(11) Publication number: **0 107 733**
**B1**

(12) **EUROPEAN PATENT SPECIFICATION**

(45) Date of publication of patent specification: **19.10.88**

(51) Int. Cl.$^4$: **C 07 C 69/24**, C 07 C 59/76, A 61 K 31/21, A 61 K 31/19, A 61 K 31/12

(21) Application number: **83902033.6**

(22) Date of filing: **06.05.83**

(86) International application number: **PCT/US83/00721**

(87) International publication number: **WO 83/04019 24.11.83 Gazette 83/27**

# (54) CYCLOALIPHATIC PHARMACEUTICAL COMPOUNDS.

(30) Priority: **06.05.82 US 375755**

(43) Date of publication of application: **09.05.84 Bulletin 84/19**

(45) Publication of the grant of the patent: **19.10.88 Bulletin 88/42**

(84) Designated Contracting States: **AT BE CH DE FR GB LI LU NL SE**

(56) References cited:
AU-B- 65 329
FR-A-2 193 612
FR-A-2 419 271
GB-A-2 040 928
US-A-1 894 460
US-A-2 811 470
US-A-3 843 712
US-A-4 000 174
US-A-4 292 432

(73) Proprietor: **CBD CORPORATION**
**10880 Wilshire Boulevard Suite 1900**
**Los Angeles, CA 90024 (US)**

(72) Inventor: **KASHA, Walter J.**
**10880 Wilshire Boulevard, Suite 1900**
**Los Angeles, CA 90024 (US)**

(74) Representative: **Werner, Hans-Karsten, Dr. et al**
**Deichmannhaus am Hauptbahnhof**
**D-5000 Köln 1 (DE)**

EP 0 107 733 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention provides compounds of the formula

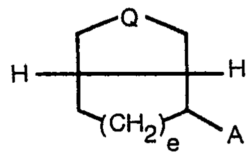

wherein,

Q is CO, CH—OR,

A is $-CRR-(CR^1R^2)_nR_3$

N is an integer of from one to eight;

R is H or lower alkyl (methyl, ethyl, propyl, butyl);

$R^1$ and $R^2$ are independently R, OR, COOH, CHO, or $CR^1R^2$ is CO;

$R_3$ is H, COOR, OR, CHR—OR or COR;

e is an integer from 1 to 2;

provided that the A group contains at least one of COOR, CHO, CO, OR and COR, and not more than one of COOH, CO or COR, and wherein an ethylenic bond may be present in the ring, or a pharmaceutically acceptable salt thereof.

As the pharmaceutically acceptable salts may be mentioned, for example, alkali metal salts, alkaline earth salts and ammoniacal salts. As specific salts may be mentioned sodium, magnesium and ammonium salts.

The cis form of such compounds constitutes a preferred aspect thereof.

In a preferred aspect, the compound (I) has the following parameters:

Q is CO, CH—OH, CH—$OCH_3$ or CH—$OCH_2CH_3$;

e is 1; and

n is 1 to 4.

A preferred group of compounds (I) has the formula

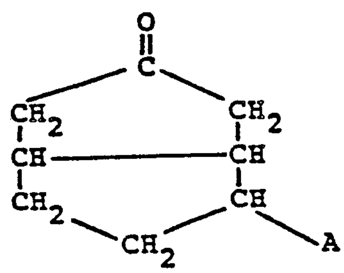

In this preferred group may be mentioned compounds, wherein A is defined by the group:

$$-CH_2-CH_2-CH_2-C(R^4R^5)R^6$$

wherein

$R^4$ and $R^5$ are independently H, methyl or ethyl;

$R^6$ is COOH, CHO or $C(R^7R^9)OR^8$; and

$R^7$, $R^8$ and $R^9$ are independently H, methyl or ethyl.

As the group $-C(R^4R^5)R^6$ may be mentioned

$-CH_2COOH$, $-CH_2CH_2OH$, $-CH_2CH(CH_3)OH$, $-CH_2CH(CH_2CH_3)OCH_3$, $-CH_2CHO$, $-CH(CH_3)CH(CH_2CH_3)OH$, or $-CH(CH_3)CH(CH_2CH_3)OCH_3$.

Particularly preferred are compounds which are 2-(5-substituted alkyl)bicyclo [3.3.0]octan-7-one, including 2-(5-methoxyhept-1-yl)bicyclo[3.3.0]octan-7-one, 2-(5-hydroxyhept-1-yl)bicyclo[3.3.0]octan-7-one, 2-(5-hydroxy-4-methylhept-1-yl)bicyclo[3.3.0]octan-7-one, and 2-(5-methoxy-4-methylhept-1-yl)bicyclo[3.3.0]-octan-7-one.

Also provided is an anti-androgenic composition suitable for providing an anti-androgenic effect when administered to a patient which comprises a pharmaceutically effective amount of a compound (I) and a pharmaceutically inert carrier suitable for topical administration of said compound to said patient. As the pharmaceutically inert carrier may be mentioned an alcohol, salve, suspension, emulsion, ointment, cream, powder or spray. In a preferred embodiment, an alcohol such as ethanol and isopropanol are preferred as a pharmaceutically inert carrier.

In a further preferred embodiment, an anti-androgenic composition with the compound (I) is provided in a sustained release vehicle for transdermal application to the skin of a patient. The sustained release medium should be one which will maintain the compound (I) at the skin and permit release to the skin for a period of preferably at least about six to about eight hours. As an example for a sustained release medium

may be mentioned polyvinylalcohol having a degree of hydrolysis of about 88% and having a molecular weight of at least about 8,000. For example, a polyvinylalcohol having a molecular weight of about 20,000 is suitable for the compound (I).

A further embodiment provides a skin preparation suitable for topical administration to a patient to be exposed to ultraviolet light which includes both the Compound (I) and an anti-ultraviolet screening agent such as paraaminobenzoic acid or cocoa butter.

A shampoo is advantageously provided for sufferers of skin problems and particularly male pattern baldness, which comprises conventional shampoo ingredients having incorporation therein the compound (I).

GB—A—2,040,928 discloses compounds which are useful as intermediates in preparing isosteres of $PGI_2$. The structural features of these compounds are mainly oxobicyclo[3.3.0]octanes that show an alkyl substitution next to a carbon atom building a carbon bridge.

GB—A—2,040,928 does not anticipate structures of compounds of the present application. All compounds published by the above mentioned disclosure contain a functional oxigenated group in the bicyclic ring-system.

Compounds published in US—A—4,292,432, US—A—4,000,174, Beilsteins Handbuch der Organischen Chemie, 4. Auflage, 3. Ergänzungswerk, 10. Band 4. Teil (pp. 2821, 2835, 2853, 2879, 2893), 4. Auflage, 3. Ergängzungswerk, 6. Band, 6. Teil (pp. 4102, 4103), 4. Auflage, 3. Ergänzungswerk, 10. Band, 1. Teil (pp. 33, 36, 49) and 4. Auflage, 2. Ergänzungswerk, 6. Band (pp. 751, 754) do not include bicyclic ring structures.

The invention provides blocking androgen receptor sites in a patient which comprises introducing to said sites the compound (I), particularly via the topical route. The growth of some cancers is related to androgen, which is alleviated by blockage of androgen receptor sites by treatment with the compound (I). Male pattern baldness is also androgen linked and treated with the compound (I) to block androgen receptor sites. The compound (I) is also useful in the prevention of keloids, and wrinkling of the skin and related conditions. Extremely minute concentrations, even dilutions exceeding 100,000:1, have been effective for these conditions. Experiments generally following the methodology of Boote et al, *Biochemica et Biophysica. Acta, 607,* pp. 145—160 (1980), have shown that while control human fibroblast cells have copious collagen production, treatment with the compound (I) provides human fibroblast cells with suppressed collagen product and with an increase by 30% over control cells. Following the Salmon clonogenic assay, *Cancer Treatment Reports, 65* p. 1 (981), the compound (I) at a level of 10 mg/ml increases the chemosensitivity to standard anti-cancer drugs tested including mitomycin C, vinblastine, vincristine, cisplatinum, actinomycin D, adriamycin, hexamethylmelamine, methyl-GAG, 5-FU, melphalan and bleomycin.

A preferred embodiment is the provision of an anti-acne treatment, where the patient afflicted with acne is treated with the compound (I), which reduces androgen to reach androgen receptor sites, thereby alleviating the acne problem.

While topical application of the compound (I) constitutes one embodiment of the invention, other routes for pharmaceutical administration are also contemplated, particularly the oral and suppository routes. Oral dosage unit formulations include tablets, capsules and other conventional oral forms. As a tablet the compound (I) is typically present in an amount of from about 1 to about 50% by weight, with the inert carrier constituting the remainder of the tablet. Tablets are compressed in a conventional manner, with typically one percent magnesium stearate being included in the mixture to be tabletted. Liquid oral dosage unit formulations may also be used in which the compound (I) is incorporated into vehicles conventionally used for lipid soluble compounds. By including at least one carboxy or carboxy ester group in the compound, a hydrophillic character may be obtained. Suppositories with the compound (I) are also contemplated, to provide a rectal suppository administration of the drug and which form takes advantage of the usual suppository ingredients.

The high potency of the compound (I) permits relatively low dosages both systematically via oral or suppository route or through topical (transdermal) applications. A concentration of the compound (I) of from about 0.001 to 5 percent by weight of the composition, and generally from about 0.01 to about one percent. Topical application on an infrequent basis, including a sustained release delivery, may indicate a relatively higher amount of the compound (I), e.g., preferably in the range of from about 0.05 to about 3 percent by weight. A relatively lower concentration of the compound (I) is indicated where a relatively larger surface area is treated, such as the back, chest, etc., e.g., a concentration of from about 0.01 to one percent by weight.

Where the compound (I) is indicated for systemic delivery, oral, injection, suppository and sublingual forms may be used, preferably the compound (I) is administered as an oral dosage unit form, such as a tablet, capsule, powder or other traditional dosage unit form. In a preferred embodiment, the oral dosage unit form is a tablet which contains a relatively small amount of the compound (I), which is due to the high potency as an anti-androgenic of the compound (I). A single oral dosage unit formulation, when administered as one oral dosage unit formulation several times per day, generally up to about four times per day, will for a normal adult male comprise an amount of about 0.0001 to about 40 mg per oral dosage unit form, and preferably from about 0.01 to about 2 mg per oral dosage unit form.

It is to be understood that the extremely small amount of the compound (I) necessarily means as a practical matter that a "normal" tablet size will have only a very small percentage of the compound (I), with

the remainder comprising pharmaceutically inert ingredients such as talcum, maize starch, polyvinyl pyrrolidone and lactose, together with a small amount of a tabletting agent such as magnesium stearate.

The effectiveness of the compound (I) is determined *in vitro* in accordance with the method of Thomas et al, *J. Clinical Endocrinology and Medicine. 38,* 19 (1974).

The general scheme for preparing the novel compounds comprises two phases. In one phase the ring structure is prepared, in the other the side chain in the form of a precursor of the desired side chain. The ring and side chain precursors are combined and subjected to further reaction to generate the desired product.

The following examples illustrate the invention:

## Example 1

Ring and Side Chain Combination:

Synthesis of 3-(5-methoxyhept-1-yl)-cyclopentene of 5-methoxyhept-1-yl Magnesium Chloride

Magnesium metal turning (7.2 g, 0.299 moles) were added to a three-neck, round-bottom flask equipped with a Friedrich condenser and kept under $N_2$ gas. Tetrahydrofuran (300 ml) was transferred to the flask and the contents allowed to stir. The clear, colorless solution of 1-chloro-5-methoxyheptane (48.1 g, 0.292 moles) was added and refluxed. The final third portion was added and allowed to stir for 12 hours. The dark yellow solution was cooled to −25°C, the condenser was removed and replaced with a dry ice addition funnel. The clear solution of 3-chloro cyclopentene (29.9 g, 0.292 moles) was added over one hour. The viscous solution was poured into two liters of $H_2O$, extracted with ether, and dried over $Na_2SO_4$. Distillation yielded 3-(5-methoxyhept-1)-yl-cyclopent-2-ene (51.5 g, 0.262 moles) as clear, colorless oil. BP. 54°C/0.1 mm.

Synthesis of 6,6-dichloro-2-(5-methoxyhept-1-yl)bicyclo-[3.2.0]heptan-7-one

A 1,000 ml three-neck, round-bottom flask was equipped with a reflux condenser 3-(5-methoxyhept-1-yl)-cyclopentene (15.0 g, .076 moles) in 300 ml of hexane. Freshly distilled dichloroacetylchloride (35.1 g, .240 moles) was added and the solution stirred via a mechanical stirrer and heated to reflux. Triethylamine (25.2 g, 0.249 moles), dissolved in 200 ml hexane, was added dropwise and the refluxing solution allowed to stir for 4 hours. The solvent was removed and the residue chromatographically purified over silica gel, leaving the product (17 g).

Synthesis of 6,6-dichloro-2-(5-methoxyhept-1-yl)bicyclo[3.3.0]-octan-7-one

The starting material 6,6-dichloro-2-(5-methoxyhept-1-yl)bicyclo (3.2.0) heptan-7-one (11 g, .036 moles) was dissolved in 100 ml of ether and transferred to a 500 ml, round-bottom glass. Excess diazomethane was generated *in situ* by reacting p-tolylsulfonylmethylnitrosamide (21.5 g) with KOH in ethanol. The $CH_2N_2$ was allowed to react for three hours, after which time acid was added to destroy any remaining diazomethane. The solution was extracted with ether and dried over $Na_2SO_4$, yielding the crude product as an orange oil (10.3 g) suitable for the next RXN, although some of the starting material was still present as evidenced by IR and gas chromatography.

Synthesis of 2-(5-methoxyhept-1-yl)bicyclo-[3.3.0]octan-7-one

To a single-neck 100 ml, round-bottom flask equipped with a condenser was added 6,6 dichloro-2-(5-methoxyheptyl)-bicyclo (3.3.0) octan-7-one (45.9 g, .149 moles). The solution was stirred via magnetic stirring and powdered zinc metal (5.2 g, .079 moles) and glacial acetic acid (10 ml, .17 moles) was added and the solution allowed to reflux for six hours, during which time white $ZnCl_2$ precipitates out of solution. The solution was filtered, washed with $NaHCO_3$ and extracted with ether three times 50 ml. The etheral extracts were combined and dried over $Na_2SO_4$. The resulting yellow oil was chromatographed with silica gel and eluted with 3:1 hexane:ether. The fractions were combined, yielding 2-(5-methoxyhept-1-yl)-bicyclo (3.3.0) octan-7-one (33 g, .13 moles) as a clear colorless oil.

## Example 2

Synthesis of 2-(5-methoxyhept-1-yl) bicyclo (3,2,0) heptan-7-one

Zinc-copper catalyst (3 g) was added to a stirred solution of 6,6-dichloro-2-(5-methoxyhept-1-yl)-bicyclo[3.2.0]-heptan-7-one (2 g, 6 mM) in acetic acid (100 ml) under a nitrogen atmosphere. The solution was stirred at room temperature for one hour, then refluxed for 1 hour, after which time the mixture was filtered through a sintered glass funnel and the etheral solution dried over $Na_2SO_4$. The solvent was removed under vacuum, leaving the crude product. Chromatography on silica gel yields the compound of the invention, 2-(5-methoxyhept-1-yl) bicyclo (3,2,0) heptan-7-one (1.2 g) which, optionally, can be used to generate another compound of the invention by reaction with diazomethane to expand the ring and form the product of Example 2.

*Analysis:* IR 2933, 2857, 2821, 1806, 1490, 1460, 1410, 1378, 1301, 1273, 1265, 1240, 1155, 1115, 1093, 939, 935, 917, 796, 746, 726, 695, 683, 664.

Example 3

Synthesis of 2-(5-hydroxyhept-1-yl) bicyclo (3,3,0) octan-7-one

2-(5-methoxyhept-1-yl) bicyclo (3,3,0) octan-7-one (1 g, 3.8 mM) (prepared as in Example 1) in ether (5 ml) was added to a solution of acetic anhydride (40 ml). The stirring mixture was cooled to 0°C and freshly distilled boron trifluoride etherate (7.0 ml) cooled to 0°C was added. The solution was stirred at 0°C for 25 hours, after which time the reaction was poured into ice water and stirred for two hours, followed by ether extraction (3×100 ml). The combined etheral extracts were washed with $NaHCO_3$ (3×100 ml), dried over $Na_2SO_4$, and the solvent removed under vacuum, leaving a yellow residue. Chromatography on silica gel yielded 2-(5-hydroxyhept-1-yl) bicyclo (3,3,0) octan-7-one (0.18 gl), the hydroxy analog of the products of examples 1 and 2.

*Analysis:* IR 3395, 2935, 2855, 1748, 1465, 1267, 1245, 1160, 1120, 965, 920, 810, 785, 745.

Examples 4—10

4. 2-(5-hydroxypent-1-yl) bicyclo [3,3,0] octan-7-one
2935, 2865, 1742, 1465, 1420, 1265, 1230, 1175, 1155, 1050, 945, 735

5. 2-(4-carboxylbut-1-yl)-bicycle [3,3,0] octan-7-one
3150, 2935, 2865, 1745, 1710, 1665, 1460, 1265, 1235, 1190, 1165, 1150, 1050, 938, 725

6. 2-(5-carboxylpent-1-yl)-bicyclo [3,3,0] octan-7-one
3100, 2935, 2860, 2835, 1743, 1705, 1660, 1465, 1260, 1225, 1170, 1155, 1045, 935

7. 2-(6-carboxylhex-1-yl)-bicycle [3,3,0] octan-7-one
3380, 2935, 2860, 1743, 1465, 1420, 1260, 1235, 1140, 1055, 935

8. 2-(3-oxopent-1-yl) bicycle [3,3,0]octan-7-one
2938, 2865, 2820, 1745, 1708, 1460, 1435, 1415, 1275, 1255, 1230, 1120, 1070, 940, 745

9. 2-(3-hydroxypent-1-yl) bicycle [3,3,0] octan-7-one
3400, 2935, 2865, 1743, 1465, 1435, 1420, 1260, 1225, 1125, 1065, 935, 735

10. 2-(5-methoxyhept-1-yl) bicycle [3,3,0] octan-7-ol
3420, 2935, 2860, 2835, 1465, 1425, 1380, 1365, 1245, 1195, 1155, 1095, 1070, 930

Example 11

In vitro experiments were conducted in a procedure modified from that disclosed by Thomas and Oake, "Androgen Metabolism in the Skin of Hirsute Women, *J.C.E. and M., 38,* 19 (1974). While compounds 9 and 10 are preferred, positive test results are shown in the table below for compounds of Formula 6, 13, 7 and 8, which were assayed using balded scalp examples:

Specific preferred compounds include the following:

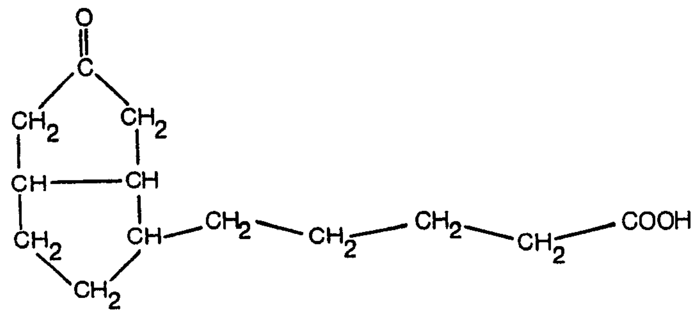

Formula 6

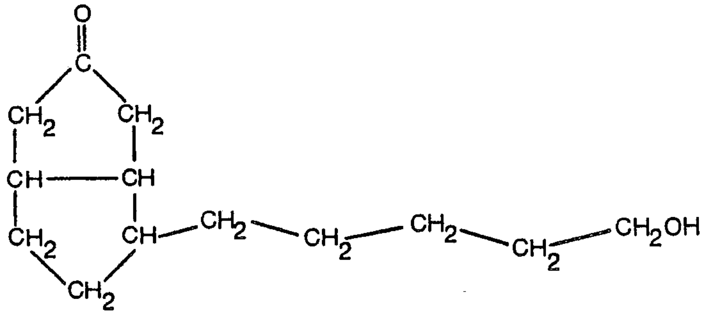

Formula 7

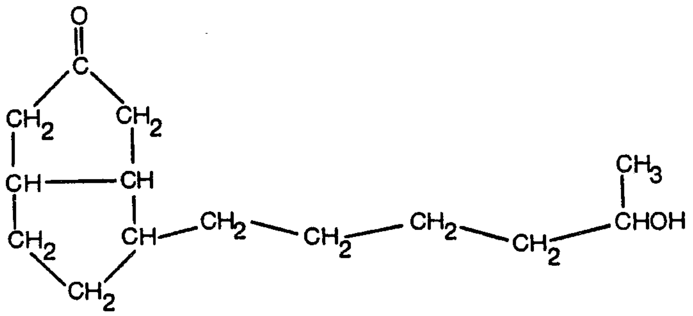

Formula 8

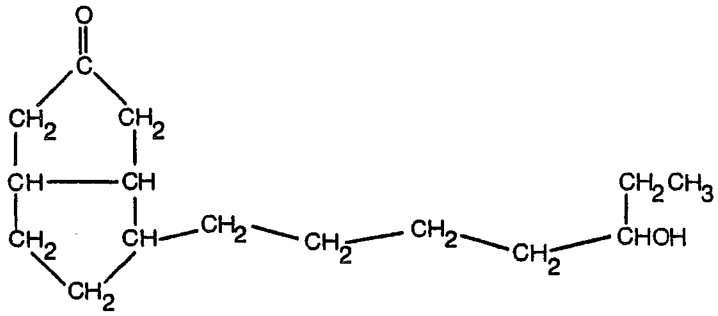

Formula 9

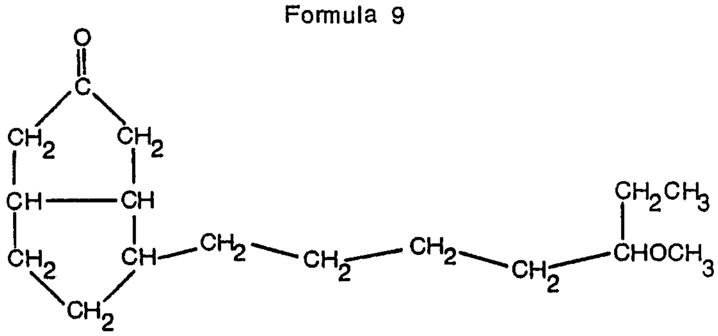

Formula 10

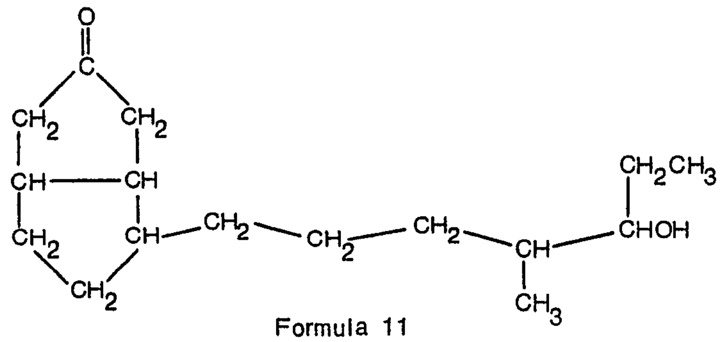

Formula 11

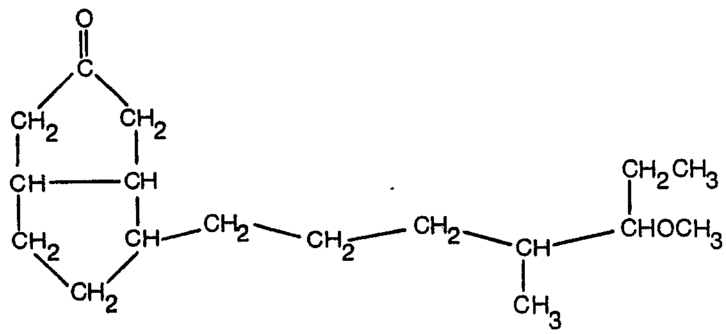

Formula 12

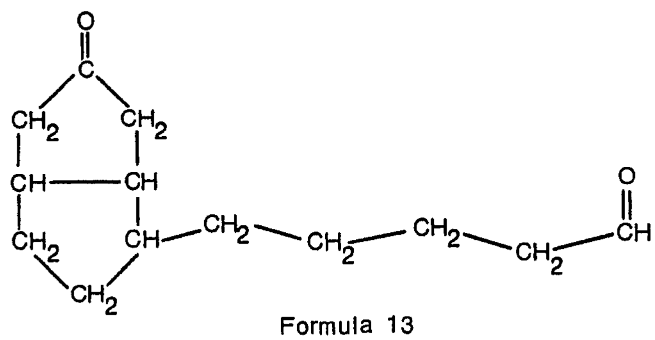

Formula 13

Percent Receptor Blocking

| Compound | Patient Number | Ratio of $^3H$ DHT to Compound 1:1 | 1:10 | 1:20 | 1:100 | 1:1000 |
|---|---|---|---|---|---|---|
| 6 | 1 | 0 | 0 | 0 | 0 | 12% |
| " | 2 | 0 | 0 | 0 | 0 | 32% |
| " | 3 | 0 | 0 | 0 | 0 | 16% |
| 13 | 1 | 0 | 0 | 0 | 0 | 15% |
| " | 2 | 0 | 0 | 0 | 0 | 30% |
| " | 3 | 0 | 0 | 0 | 0 | 10% |
| 7 | 1 | 0 | 0 | 18 | 30 | 36% |
| " | 2 | 0 | 0 | 24 | 35 | 42% |
| " | 3 | 0 | 3 | 33 | 44 | 51% |
| 8 | 1 | 0 | 0 | 17 | 33 | 37% |
| " | 2 | 0 | 0 | 22 | 38 | 41% |
| " | 3 | 0 | 0 | 34 | 49 | 52% |

Example 12

Folowing the procedure of Example 11, compounds of Formulae 9 and 10 were tested to determine their percent inhibition of labeled androgen binding to the skin, the test utilizing caesarean section skin from hirsuite patients:

| Ratio of compound to androgen | % inhibition of $^3H$ Androgen binding to skin Compound 9 | Compound 10 |
|---|---|---|
| 10 : 1 | 25% | 32% |
| 100 : 1 | 51% | 55% |
| 1000 : 1 | 75% | 80% |
| 10000 : 1 | 83% | 86% |

In skin samples from hirsuite patients the compounds of this invention blocked the binding $^3H$ DHT to the androgen receptor site. DHT was selected for use in the assay because it is a more potent androgen than testosterone and exhibits a greater (stronger) affinity for the receptor site. Furthermore, there is evidence that 5 alpha reductase enzumes converts testosterone to DHT *in vivo,* and it is DHT which is the active steroid on the *in vivo* receptor sites.

In order to substantiate the effects of the derived anti-androgen compounds on actual balded scalp specimens, skin was obtained from volunteers undergoing hair transplantation. The bald skin was used for androgen receptors using the second technique described above. The androgen used was dihydrotestosterone (DHT) vs. the anti-androgen compounds afore herein recited.

The results with compounds 9 and 10 are shown in Table below.

% inhibition of Binding $^3H$ DHT to Androgen Receptor

COMPOUND 9

| PATIENT | ratio of compound 9:$^3H$ DHT | | | | |
|---|---|---|---|---|---|
| # | 10:1 | 20:1 | 100:1 | 200:1 | 1000:1 |
| #1 | 25% | 30% | 35% | 39% | 42% |
| #2 | 40% | 47% | 69% | 72% | 75% |
| #3 | 0% | 0% | 13% | 15% | 22% |
| #4 | 14% | 43% | 45% | 47% | 50% |
| #5 | 17% | 46% | 46% | 48% | 50% |
| #6 | 15% | 36% | 37% | 39% | 43% |

| PATIENT | ratio of compound 10:$^3H$ DHT | | | | |
|---|---|---|---|---|---|
| # | 10:1 | 20:1 | 100:1 | 200:1 | 1000:1 |
| #1 | 50% | 60% | 67% | 68% | 70% |
| #2 | 43% | 59% | 74% | 76% | 77% |
| #3 | 0% | 0% | 17% | 18% | 25% |
| #4 | 19% | 47% | 47% | 48% | 50% |
| #5 | 23% | 51% | 51% | 52% | 54% |
| #6 | 18% | 41% | 42% | 45% | 50% |

Again, the nonhirsuite patient (having less androgenic sites available for interaction) displayed the preferred uptake for the natural androgen over any of the new family of anti-androgens. This data supports the concept of higher $K_d$ for these compounds compared with that of the natural androgen:

% Inhibition of Binding 3H DHT to Androgen Receptor

| | Ratio of Compound to $^3H$ DHT | | | |
|---|---|---|---|---|
| Compound | 10:1 | 100:1 | 1000:1 | 10000:1 |
| 9 | 52 | 60 | 64 | 68 |
| 10 | 40 | 47 | 56 | 65 |

Competitive inhibition experiments were performed using compound 9 in the second technique described above. The results are presented in the tables below.

As noted below, the test compound inhibited the androgen receptor site at 10 molecules of compound 9 or of compound 10 for one molecule of testosterone. The degree of inhibition increased as the ratio of compound/androgen increased.

The *in vitro* interaction of compounds 9 and 10 with dihydrotestosterone are recorded below.

The ratio of 10 or 20 to 1 is readily exceeded by topical therapy, as the ratio of compound 9 to androgen applied topically in the small therapeutic trial was in excess of 10,000 to 1. This lipophilic molecule would be concentrated in the pilosebaceous glands.

*In vitro* inhibition of the Androgen receptor-DHT interaction of Compound 9 and Compound 10

| Ratio of Compound to Androgen | Femtamoles of DHT bound/mg protein* | % of Androgen receptor inhibition |
|---|---|---|
| Compound 9 0:1 | 78 | 0 |
| 10:1 | 58 | 25.6 |
| 100:1 | 46 | 41.0 |
| Compound 10 0:1 | 78 | 0 |
| 10:1 | 44 | 43.6 |
| 100:1 | 38 | 51.3 |

*In vitro* inhibition of the Androgen receptor-testosterone interaction by Compound 9 and Compound 10

| Ratio of Compound to Androgen | Femtamoles of testosterone bound/mg protein* | % of Androgen receptor inhibition |
|---|---|---|
| Compound 9 0:1 | 3.6 | 0 |
| 20:1 | 2.0 | 43.7% |
| Compound 10 0:1 | 3.6 | 0 |
| 20:1 | 1.9 | 47.0 |

*Corrected for non-specific binding

Example 13

*In vitro* studies utilizing $^3H$ steroids and anti-androgen compound to demonstrate that the subject anti-androgen(s) do not displace any of the natural occurring hormone away from SHBG (Sex Hormone Binding Globulin).

| SHBG plus the following: | cpm |
|---|---|
| DHT | 3900 counts |
| DHT + $10^4$ x Compound 11 | 3890 |
| DHT + $10^4$ x Compound 12 | 3910 |
| Estradiol | 2100 |
| Estradiol + $10^4$ x Compound 11 | 2130 |
| Estradiol + $10^4$ x Compound 12 | 2071 |
| Progesterone | 1840 |
| Progesterone + $10^4$ x Compound 11 | 1800 |
| Progesterone + $10^4$ x Compound 12 | 1900 |
| Testosterone | 2600 |
| Testosterone + $10^4$ x Compound 11 | 2550 |
| Testosterone + $10^4$ x Compound 12 | 2570 |

Example 14

Inhibition of the androgen receptor and DHT in six representative patient samples are noted below:

RATIO OF COMPOUND 11 to DHT

| | Patient | 10 | 20 | 100 | 200 | 1000 |
|---|---|---|---|---|---|---|
| % INHIBITION | 1 | 25 | 30 | 35 | 39 | 42 |
| OF | 2 | 40 | 47 | 69 | 72 | 75 |
| RECEPTOR SITE | 3 | 0 | 0 | 13 | 15 | 22 |
| | 4 | 14 | 43 | 45 | 47 | 50 |
| | 5 | 17 | 46 | 46 | 49 | 50 |
| | 6 | 15 | 36 | 37 | 39 | 43 |
| MEAN % INHIBITION | | 18.5 | 33.7 | 40.8 | 43.5 | 47.0 |
| | S.D. | 13.3 | 17.7 | 18.2 | 18.5 | 17.1 |

Example 15

Inhibition of the androgen receptor and DHT in six representative patient samples are noted below for Compound 12:

RATIO OF COMPOUND 12 to DHT

| | Patient | 10 | 20 | 100 | 200 | 1000 |
|---|---|---|---|---|---|---|
| % INHIBITION | 1 | 50 | 60 | 67 | 68 | 70 |
| OF | 2 | 43 | 59 | 74 | 76 | 77 |
| RECEPTOR SITE | 3 | 0 | 0 | 17 | .18 | 25 |
| | 4 | 19 | 47. | 47 | 48 | 50 |
| | 5 | 23 | 51 | 51 | 52 | 54 |
| | 6 | 18 | 41 | 42 | 45 | 50 |
| MEAN % INHIBITION | | 25.5 | 43.0 | 49.7 | 51.2 | 54.3 |
| | S.D. | 18.2 | 22.3 | 20.1 | 20.2 | 18.2 |

Example 16

Wrinkling of the skin involves the decreased formation of elastin and the increased formation of collagen by supporting cells, predominantly the fibroblasts. Experiments show that the control (non-treated) cells had only trace elastin production but copious collagen production. The cells treated with the compound of Formula 10 had approximately 30% elastin production with a resultant decrease in collagen production.

Example 17

Collagen formation has been found to be decreased in experiments with compounds of Formulas 6 through 13 inclusive, whilst showing a massive observable increase in the formation of elatin. It is to be recognized that periperhal effects, such as the arterial and dermial, when affected without altering more basic hormonal essential functions of the androgens, provides a beneficial result.

Example 18

The following results wee observed at concentrations of anti-androgen of formula 10 to DHT of 1000:1.

| Patient | % of Collagen synthesized | Fold Elastic increase compared to Control |
|---|---|---|
| 1 | 23 | 1.4 |
| 2 | 23 | 4.7 |
| 3 | 54 | 2 |
| 4 | 43 | 4 |
| 5 | 59 | 6 |
| 6 | 63 | 2 |
| 7 | 39 | 3 |
| 8 | 41 | 3 |
| 9 | 66 | 4 |
| 10 | 57 | 6 |
| 11 | 39 | 3 |

These quantitative experiments demonstrate the importance of the anti-androgen and its therapeutic use in the topical application for alterning collagen:elastin formation, thus decreasing wrinkles; but more importantly, its systemic applications would include its use in decreasing the rate of atheroschlerosis by decreasing the rate of collagen formation.

Example 19

The anti-cancer activity of the Compound (I) was tested on tumors which had been obtained after surgery which were placed in an appropriate testing media. Samples of cancers from the prostate, ovary and breast were tested, with a recognition that these cells lines have a high level of androgen binding. The *in vitro* clonogenic assay of Salmon et al, *New England Journal of Medicine, 298* 1321 (1978) was used. The Compound (I) was shown to have a significant anti-tumor activity against the three selected tumors when used at a level of *10mcg/ml* with the compound of the Formula 10, based upon observations of a cytologist.

The Compound (I) compares favourably in tests against mitomycin C, vinblastine, vincristine, melamine, methyl-CAG, 5-FU, melphalan, and bleomycin. The relationship of the androgen blockage to the anticancer applicability of the Compound (I) is seen by the more pronounced effects that were observed on prostatic cancer, as if the prostate has a greater number of androgen receptor sites than the other tested cancers.

Thre prostatic cancers and six specimens of benign prostatic hypertrophy were used for experiments, with androgen receptors being found in all. The compound of the Formula 10 blocked between 35 and 87% of the hormonal androgen receptors at 1:1000 DHT to the Compound (I), and no significant deleterious effects were noticed in the estrogen and progesterone receptors.

Example 20

The compound of Formula 10 was found to block 59—80% of androgen binding sites in a melanoma at concentrations of 1:1000 of dihydrotestosterone to the tested compound, with no significant effect on either the estrogen or progesterone receptors in the tissue.

Example 21

The compound of Formula 8 was tested on tumor cells in the absence of steroid, with squamous skin cancer cells being employed for assays on this compound. The test results showed that there was no effect observed on cell counts or viability and that there is a concentration-dependent decrease in $^3$H thymidine update. The tests which were conducted show that Compound (I) has activity against cancer cells, even those with non-hormone receptors, suggesting the use of the Compound (I) for therapy for numerous forms of cancer, singularly, and/or in conjunction with present standard chemotherapies.

Example 22

Lou Gehrig's disease, amyotrophic laterial sclerosis, is advantageously treated with the Compound (I), based upon tests with surgical specimens of neurotissues with this disease. The compound of Formula 10 blocked 79% of the androgen receptors in these tissues of both benign and malignant tissues at ratios of DHT to the tested compound of 1:1000. There was no measurable estrogen receptor inhibition in this test, and there was only 3% progesterone receptor inhibition.

Astrocytoma tissue was similarly treated with 21% androgen site blockage and the same results for estrogen and progesterone receptor inhibition. Gliablastoma tissue showed 39% androgen receptor inhibition, no estrogen receptor inhibition, and 2% progesterone inhibition. Meningioma tissue showed 64% androgen receptor inhibition, no estrogen receptor inhibition, and 5% progesterone receptor inhibition.

Example 23

Keloid formation is suppressed by blocking androgen receptor sites. In its generic aspect, the invention comprises a method of suppressing the formation of keloids which comprises topically applying to a patient an anti-androgenic agent to the skin of said patient, whereby androgen receptor sites are blocked, thereby retarding the formation of keloids. Tests conducted with 2-(5-methoxyhept-1-yl)-bicyclo-[3.3.0]octan-7-one have shown the suppression of keloid formation. Surgical excision of keloids was performed from clinic and private patient populations. 2-(5-methoxyhept-1-yl)-bicyclo-[3.3.0]octan-7-one was incubated in ratios of 1:0 to 1:10000 steroid hormone to this compound. Non-specific binding was determined with a 200-fold excess of unlabeled ligand at each concentration of $^3$H-ligand use. Male patients showed neither detectable estrogen or progesterone binding. Mean DHT binding was 867 femtamoles/mg cytosalprotein ±101 femtamoles/mg. The following results were obtained:

| Pt | Sex | Race | Age | Location of Keloid | Binding of DHT | % Block | Binding of Estrogen | Binding of Progesterone |
|---|---|---|---|---|---|---|---|---|
| 1 | F | Blk | 37 | Suprapubic | 703 | 43 | 9 | 7 |
| 2 | F | Blk | 21 | Chest | 816 | 24 | 4 | Non-detectable |
| 3 | M | Cauc | 23 | Neck | 795 | 57 | Non-detectable | Non-detectable |
| 4 | M | Blk | 26 | Inguinal groin | 938 | 86 | Non-detectable | Non-detectable |

Treatment for prevention of interabdominal and other post surgical systemic adhesions were also evaluated. Skin fibroblast collagen suppression was also considered using surgical explants. At ratios of DHT: 2-(5-methoxyhept-1-yl)-bicyclo-[3.3.0]octan-7-one of 1:1000, in more than 400 experiments, the 2-(5-methoxyhept-1-yl)-bicyclo-[3.3.0]octan-7-one blocked between 22% and 94% of the androgen receptors without significant interference with either the estrogen or progesterone receptors. Tissue from keloid showed androgen receptor inhibition of 41 to 72% and no measurable estrogen or progesterone receptor inhibition. Scalp samples showed from 22 to 89% androgen inhibition and zero to 7% progesterone inhibition. Forearm sample showed 22% androgen receptor inhibition and 4% progesterone receptor inhibition. In none of the tests was there any measurable estrogen receptor inhibition.

"Hard fibrous band" formation was retarded around breast implants after augmentation surgery. Blockage of 34% to 79% of androgen receptor sites was measured for 2-(5-methoxyhept-1-yl)-bicyclo-[3.3.0]octan-7-one without any significant effects on either estrogen or progesterone binding sites. The amount protein (in mg) per gram of gross tissue was measured for four breast capsule group numbers which showed:

| Mean % Androgen Binding | Mean % Androgen Inhibition |
|---|---|
| 10.7 | 17 |
| 31.0 | 49 |
| 99.9 | 70 |
| 109.8 | 74 |

In a further embodiment there are provided compounds of the formula:

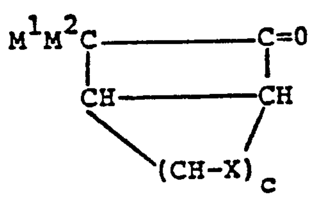

(II)

wherein

each X is independently H or A;

each M is independently H, Cl, Br or I;

A is —CRR—$(CR^1R^2)_nR_3$;

N is an integer of from one to eight;

R is H or lower alkyl;

$R^1$ or $R^2$ are independently R, OR, COOH, CHO or $CR^1R^2$ is CO;

$R_3$ is H, COOR, OR, CHR—OR or COR;

c is three or four;

provided that at least one A group is present and each A group contains at least one of COOR, CHO, CO, OR and COR, and not more than one of COOH, CO or COR, and wherein up to two ethylenic bonds may be present in the ring, or a pharmaceutically acceptable salt thereof. Preferably c is three, thus providing a bicyclo[3,2,0]heptan-7-one ring structure, and in a preferred embodiment there is only one X substituent which is at the 2 or 4 position of the alkylbicyclo[3,2,0]heptan-7-one ring. The A group is preferably

$—CH_2—CH_2—CH_2—C(R^4R^5)R^6$ wherein

$R^4$ and $R^5$ are independently H, methyl or ethyl; and

$R^6$ is COOH, CHO or $C(R^7R^9)OR^8$; and

$R^7$, $R^8$ and $R^9$ are independently H, methyl or ethyl.

The compound preferably contains a cis bridge. The group $—C(R^4R^5)R^6$ is preferably

$—CH_2COOH$, $—CH_2CH_2OH$, $—CH_2CH(CH_3)OH$, $—CH_2CH(CH_2CH_3)OCH_3$, $—CH_2CHO$, $—CH(CH_3)CH(CH_2CH_3)OH$, or $—CH(CH_3)CH(CH_2CH_3)OCH_3$.

The compounds (II) have the same utility as the compounds (I) and in some cases the compounds (II) serve as intermediates for the production of the compounds (I). For example, the compound 6,6-dichloro-2-(5-methoxyhept-1-yl)-bicyclo[3,2,0]heptan-7-one is both an intermediate for producing a compound of the formula (I) and is also itself an anti-androgenic agent sharing the utilities of the compound (I). Representative compounds (II) include:

6,6-dichloro-2-(5-hydroxyhept-1-yl)-bicyclo[3,2,0]heptan-7-one
2-(5-methoxyhept-1-yl)-bicyclo[3,2,0]heptan-7-one
6-chloro-2-(5-methoxyhept-1-yl)-bicyclo[3,2,0]heptan-7-one
6,6-dichloro-4-(5-methoxyhept-1-yl)-bicyclo[3,2,0]heptan-7-one
6-chloro-2-(5-methoxyhept-1-yl)-bicyclo-[3.2.0]heptan-7-one
6-chloro-2-(5-hydroxyhex-1-yl)-bicyclo-[3.2.0]heptan-7-one
6,6-dichloro-4-(5-hydroxyhept-1-yl)-bicyclo[3,2,0]heptan-7-one

The compounds (II) are useful in anti-androgenic composition suitable for providing an anti-androgenic effect when administered to a patient in the same manner as for compound (I). A skin preparation is provided which is suitable for topical administration to a patient to be exposed to ultraviolet light which includes an anti-ultraviolet screening agent.

**Claims for the Contracting States: BE CH DE FR GB LI LU NL SE**

1. A compound of the formula

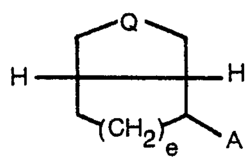

wherein

Q is CO, CH—OR,

A is $—CRR—(CR^1R^2)_nR_3$,

n is an integer of from one to eight;

R is H or lower alkyl (methyl, ethyl, propyl, butyl);

$R^1$ and $R^2$ are independently R, OR, COOH, CHO, or $CR^1R^2$ is CO;

$R_3$ is H, COOR, OR, CHR—OR or COR;

e is an integer from 1 to 2;

provided that the A group contains at least one of COOR, CHO, CO, OR and COR, and not more than one of COOH, CO or COR, and wherein an ethylenic bond may be present in the ring, or a pharmaceutically acceptable salt thereof.

2. A compound of claim 1, wherein Q is CO, CH—OH, $CH—OCH_3$ or $CH—OCH_2CH_3$; e is 1; and n is 1 to 4.

3. A compound of claim 1, wherein said compound has the formula

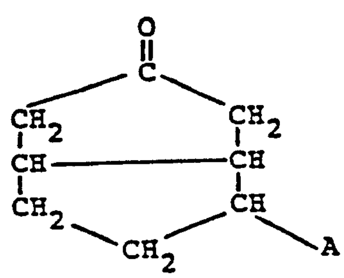

4. A compound of claim 3, wherein A has the formula

$$—CH_2—CH_2—CH_2—C(R^4R^5)R^6$$

wherein

$R^4$ and $R^4$ are independently H, methyl or ethyl;

$R^6$ is COOH, CHO or $C(R^7R^9)OR^8$; and

$R^7$, $R^8$ and $R^9$ are independently H, methyl or ethyl, and the compound contains a cis bridge.

5. A compound of claim 4, wherein $—C(R^4R^5)R^6$ is
$—CH_2COOH$, $—CH_2CH_2OH$, $—CH_2CH(CH_3)OH$, $—CH_2CH(CH_2CH_3)OCH_3$, $—CH_2CHO$, $—CH(CH_3)CH(CH_2CH_3)OH$, or $—CH(CH_3)CH(CH_2CH_3)OCH_3$.

6. A compound of claim 1 which is 2-(5-methoxyhept-1-yl)-bicyclo-[3.3.0]octan-7-one.

7. A compound of claim 1 which is 2-(5-hydroxyhept-1-yl)-bicyclo-[3.3.0]octan-7-one.

8. A compound of claim 1 which is 2-(5-hydroxy-4-methylhept-1-yl)-bicyclo-[3.3.0]octan-7-one.

9. A compound of claim 1 which is 2-(5-methoxy-4-methylhept-1-yl)-bicyclo-[3.3.0]octan-7-one.

10. A composition comprising a pharmaceutically effective amount of a compound of any of claims 1—9.

11. The composition of claim 10 comprising an inert carrier suitable for topical administration of said compound to said patient.

12. The composition of claim 11, wherein said pharmaceutically inert carrier includes an alcohol, salve, suspension, emulsion, ointment, cream, powder or spray.

13. The composition of claim 11, wherein said pharmaceutically inert carrier includes an alcohol.

14. The composition of any of claims 10 to 13 which is contained in a sustained release medium, whereby upon application of said sustained release medium the composition is released to the skin over a prolonged period of time.

15. The composition of any of claims 10 to 13 in the form of a suppository for rectal administration.

16. The composition of any of claims 10 to 13 which is suitable for topical administration including an anti-ultraviolet screening agent.

17. A shampoo which includes the composition of any of claims 10 to 13.

18. A compound of the formula

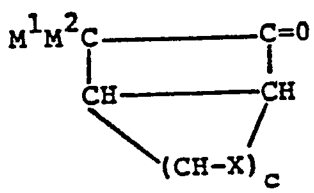

(II)

wherein

each X is independently H or A;

each M is independently H, Cl, Br, or I;

A is $—CRR—(CR^1R^2)_nR_3$;

n is an integer of from one to eight;

R is H or lower alkyl;

$R^1$ and $R^2$ are independently R, OR, COOH, CHO, or $CR^1R^2$ is CO;

$R_3$ is H, COOR, OR, CHR—OR or COR;

c is three or four;

provided that at least one A group is present and each A group contains at least one of COOR, CHO, CO, OR and COR, and not more than one of COOH, CO or COR, and wherein up to two ethylenic bonds may be present in the ring, or a pharmaceutically acceptable salt thereof.

19. A compound of claim 18, wherein c is three, thus constituting a bicyclo[3,2,0]heptan-7-one ring structure.

20. A compound of claim 19, which contains one X substituent which is at the 2 or 4 position of the alkylbicyclo[3,2,0]heptan-7-one ring.

21. A compound of claim 20 wherein A is

$$—CH_2—CH_2—CH_2—C(R^4R^5)R^6$$

wherein

$R^4$ and $R^5$ are independently H, methyl or ethyl;

$R^6$ is COOH, CHO or $C(R^7R^9)OR^8$; and

$R^7$, $R^8$ and $R^9$ are independently H, methyl or ethyl, and the compound contains a cis bridge.

22. A compound of claim 21, wherein $—C(R^4R^5)R^6$ is
$—CH_2COOH$, $—CH_2CH_2OH$, $—CH_2CH(CH_3)OH$, $—CH_2CH(CH_2CH_3)OCH_3$, $—CH_2CHO$, $—CH(CH_3)CH(CH_2CH_3)OH$, or $—CH(CH_3)CH(CH_2CH_3)OCH_3$.

23. A compound of claim 18 which is 6,6-dichloro-2-(5-methoxyhept-1-yl)-bicyclo[3,2,0]heptan-7-one.

**Claims for the Contracting State: AT**

1. Process for preparing a compound of the formula

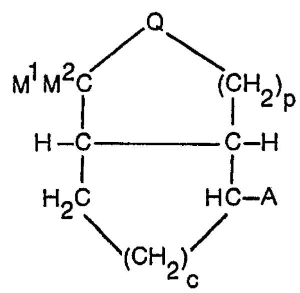

(1)

wherein

$M^1$ and $M^2$ are hydrogen;

Q is CO, CH—OR, or —CR(OH);

A is $—CRR—(CR^1R^2)_nR_3$,

n is an integer of from one to eight;

R is H or lower alkyl (methl, ethyl, propyl, butyl);

$R^1$ and $R^2$ are independently R, OR, COOH, CHO, or $CR^1R^2$ is CO;

$R_3$ is H, COOR, OR, CHR—OR or COR;

e is an integer from 1 to 2; and

p is zero or one:

provided that the A group contains at least one of COOR, CHO, CO, OR and COR, and not more than one of COOH, CO or COR, and wherein an ethylenic bond may be present in the ring, or a pharmaceutically acceptable salt thereof which comprises dehalogenating a dihalide of formula I in which $M^1$ and $M^2$ are halogen all other symbols being defined as above.

2. The process of claim 1 in which zinc is used as a dehalogenation catalyst in an acid medium.

3. Process for preparing compounds of claim 1 or 2 and formula I, wherein Q is CO, CH—OH, $CH—OCH_3$ or $CH—OCH_2CH_3$; c is 1; and n is 1 to 4.

4. A process of claim 1, wherein said compound has the formula

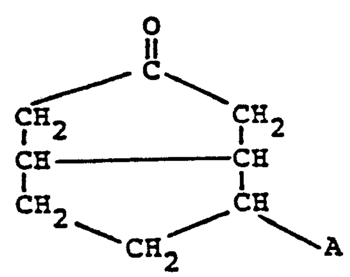

5. A process of claim 4, wherein A has the formula

$$—CH_2—CH_2—CH_2—C(R^4R^5)R^6$$

wherein

$R^4$ and $R^5$ are independently H, methyl or ethyl;

$R^6$ is COOH, CHO or $C(R^7R^9)OR^8$; and

$R^7$, $R^8$ and $R^9$ are independently H, methyl or ethyl, and the compound contains a cis bridge.

6. A process of claim 5, wherein $—C(R^4R^5)R^6$ is

$—CH_2COOH$, $—CH_2CH_2OH$, $—CH_2CH(CH_3)OH$, $—CH_2CH(CH_2CH_3)OCH_3$, $—CH_2CHO$, $—CH(CH_3)CH—(CH_2CH_3)OH$, or $—CH(CH_3)—CH(CH_2CH_3)OCH_3$.

7. A process of claim 1 wherein the compound is 2-(5-methoxyhept-1-yl)-bicyclo-[3.3.0]octan-7-one.

8. A process of claim 1 wherein the compound is 2-(5-hydroxyhept-1-yl)-bicyclo-[3.3.0]octan-7-one.

9. A process of claim 1 wherein the compound is 2-(5-hydroxy-4-methylhept-1-yl)-bicyclo-[3.3.0]octan-7-one.

10. A process of claim 1 wherein the compound is 2-(5-methoxy-4-methyl(hept-1-yl)-bicyclo-[3.3.0]octan-7-one.

**Patentansprüche für die Vertragsstaaten: BE CH DE FR GB LI LU NL SE**

1. Verbindung der Formel

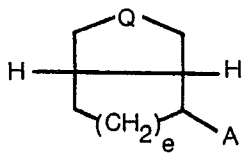

in der
Q CO, CH—OR ist,
A —CRR—$(CR^1R^2)_nR_3$ ist,
n eine ganze Zahl von eins bis acht ist,
R H oder Niederalkyl (Methyl, Ethyl, Propyl, Butyl) ist,
$R^1$ und $R^2$ unabhängig voneinander R, OR, COOH, CHO sind oder $CR^1R^2$ CO ist,
$R_3$ H, COOR, OR, CHR—OR oder COR ist,
e eine ganze Zahl von 1 bis 2 ist,
mit der Maßgabe, daß die Gruppe A wenigstens eine der Gruppierungen COOR, CHO, CO, OR und COR und nicht mehr als eine der Gruppierungen COOH, CO oder COR enthält, und worin eine ethylenische Bindung im Ring vorhanden sein kann, oder pharmazeutisch annehmbares Salz derselben.

2. Verbindung nach Anspruch 1, worin Q CO, CH—OH, CH—$OCH_3$ oder CH—$OCH_2CH_3$ ist, e 1 ist und n 1 bis 4 ist.

3. Verbindung nach Anspruch 1, worin die Verbindung die Formel

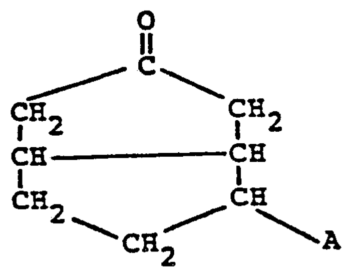

besitzt.

4. Verbindung nach Anspruch 3, worin A die Formel

$$—CH_2—CH_2—CH_2—C(R^4R^5)R^6$$

besitzt, in der
$R^4$ und $R^5$ unabhängig voneinander H, Methyl oder Ethyl sind,
$R^6$ COOH, CHO oder $C(R^7R^9)OR^8$ ist und
$R^7$, $R^8$ und $R^9$ unabhängig voneinander H, Methyl oder Ethyl sind, und die Verbindung eine cis-Brücke besitzt.

5. Verbindung nach Anspruch 4, worin $C(R^4R^5)R^6$
$—CH_2COOH$, $—CH_2CH_2OH$, $—CH_2CH(CH_3)OH$, $—CH_2CH(CH_2CH_3)OCH_3$, $—CH_2CHO$,
$—CH(CH_3)CH(CH_2CH_3)OH$ oder $—CH(CH_3)CH(CH_2CH_3)OCH_3$ ist.

6. Verbindung nach Anspruch 1, die 2-(5-Methoxyhept-1-yl)-bicyclo[3.3.0]octan-7-on ist.

7. Verbindung nach Anspruch 1, die 2-(5-Hydroxyhept-1-yl)-bicyclo[3.3.0]octan-7-on ist.

8. Verbindung nach Anspruch 1, die 2-(5-Hydroxy-4-methylhept-1-yl)-bicyclo[3.3.0]octan-7-on ist.

9. Verbindung nach Anspruch 1, die 2-(5-Methoxy-4-methylhept-1-yl)-bicyclo[3.3.0]octan-7-on ist.

10. Pharmazeutische Zusammensetzung, umfassend eine pharmazutisch wirksame Menge einer Verbindung nach irgendeinem der Ansprüche 1 bis 9.

11. Zusammensetzung nach Anspruch 10, umfassend einen inerten Träger, der für die topische Anwendung der Verbindung bei einem Patienten geeignet ist.

12. Zusammensetzung nach Anspruch 11, worin der pharmazeutisch inerte Träger einen Alkohol, eine Salbe, eine Suspension, eine Emulsion, ein Unguentum, eine Creme, ein Pulver oder ein Spray einschließt.

13. Zusammensetzung nach Anspruch 11, worin der pharmazeutisch inerte Träger einen Alkohol einschließt.

14. Zusammensetzung nach irgendeinem der Ansprüche 10 bis 13, die in einem Medium zur Langzeit-Wirkstoff-Abgabe enthalten ist, wodurch bei Applikation des Mediums zur Langzeit-Wirkstoff-Abgabe die Zusammensetzung über einen verlängerten Zeitraum an die Haut abgegeben wird.

15. Zusammensetzung nach irgendeinem der Ansprüche 10 bis 13 in Form eines Suppositoriums für die rektale Applikation.

16. Zusammensetzung nach irgendeinem der Ansprüche 10 bis 13, die für die topische Verabreichung

geeignet ist, enthaltend ein Schutzmittel gegen ultraviolette Strahlung.

17. Shampoo, enthaltend die Zusammensetzung nach irgendeinem der Ansprüche 10 bis 13.

18. Verbindung der Formel

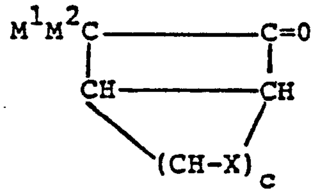

(II)

in der

X jeweils unabhängig H oder A ist,

M jeweils unabhängig H, Cl, Br oder I ist,

A $-CRR-(CR^1R^2)_nR_3$ ist,

n eine ganze Zahl von eins bis acht ist,

R H oder Niederalkyl ist,

$R^1$ und $R^2$ unabhängig voneinander R, OR, COOH, CHO sind oder $CR^1R^2$ CO ist,

$R_3$ H, COOR, OR, CHR—OR oder COR ist,

c drei oder vier ist

mit der Maßgabe, daß wenigstens eine Gruppe A vorhanden ist und jede Gruppe A wenigstens eine der Gruppierungen COOR, CHO, CO, OR und COR und nicht mehr als eine der Gruppierungen COOH, CO oder COR enthält, und worin bis zu zwei ethylenische Bindungen im Ring vorhanden sein konnen, oder pharmazeutisch annehmbares Salz derselben.

19. Verbindung nach Anspruch 18, worin c drei ist, wodurch eine Bicyclo[3.2.0]heptan-7-on-Ring-Struktur gebildet wird.

20. Verbindung nach Anspruch 19, die einem Substituenten X enthält, der sich in der 2- oder 4-Stellung des Alkylbicyclo[3.2.0]heptan-7-on-Rings befindet.

21. Verbindung nach Anspruch 20, worin A

$$-CH_2-CH_2-CH_2-C(R^4R^5)R^6$$

ist, worin

$R^4$ und $R^5$ unabhängig voneinander H, Methyl oder Ethyl sind,

$R^6$ COOH, CHO oder $C(R^7R^9)OR^8$ ist und

$R^7$, $R^8$ und $R^9$ unabhängig voneinander H, Methyl oder Ethyl sind, und die Verbindung eine cis-Brücke besitzt.

22. Verbindung nach Anspruch 21, worin $C(R^4R^5)R^6$

$-CH_2COOH$, $-CH_2CH_2OH$, $-CH_2CH(CH_3)OH$, $-CH_2CH(CH_2CH_3)OCH_3$, $-CH_2CHO$, $-CH(CH_3)CH(CH_2CH_3)OH$ oder $-CH(CH_3)CH(CH_2CH_3)OCH_3$ ist.

23. Verbindung nach Anspruch 18, die 6,6-Dichloro-2-(5-methoxyhept-1-yl)-bicyclo[3.2.0]heptan-7-on ist.

**Patentansprüche für den Vertragsstaat: AT**

1. Verfahren zur Herstellung einer Verbindung der Formel

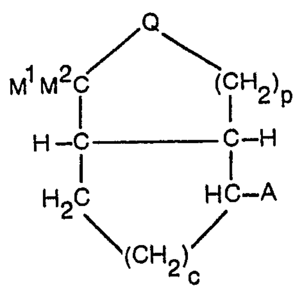

(I)

in der

$M^1$ und $M^2$ Wasserstoff sind,

Q CO, CH—OR oder —CR(OH) ist,

A $-CRR-(CR^1R^2)_nR_3$ ist,

n eine ganze Zahl von eins bis acht ist,

R H oder Niederalkyl (Methyl, Ethyl, Propyl, Butyl) ist,

$R^1$ und $R^2$ unabhängig voneinander R, OR, COOH, CHO sind oder $CR^1R^2$ CO ist,
$R_3$ H, COOR, OR, CHR—OR oder COR ist,
e eine ganze Zahl von 1 bis 2 ist und
p null oder eins ist,
mit der Maßgabe, daß die Gruppe A wenigstens eine der Gruppierungen COOR, CHO, CO, OR und COR und nicht mehr als eine der Gruppierungen COOH, CO oder COR enthält, und worin eine ethylenische Bindung im Ring vorhanden sein kann, oder eines pharmazeutisch annehmbaren Salzes derselben, umfassend das Enthalogenieren eines Dihalogenids der Formel I, in der $M^1$ und $M^2$ Halogen sind und alle anderen Symbole die im Vorstehenden angegebenen Bedeutungen haben.

2. Verfahren nach Anspruch 1, bei dem Zink als Enthalogenierungs-Katalysator in einem Säure-Medium verwendet wird.

3. Verfahren zur Herstellung von Verbindungen nach Anspruch 1 oder 2 und der Formel 1, worin Q CO, CH—OH, $CH—OCH_3$ oder $CH—OCH_2CH_3$ ist, c 1 ist und n 1 bis 4 ist.

4. Verfahren nach Anspruch 1, worin die Verbindung die Formel

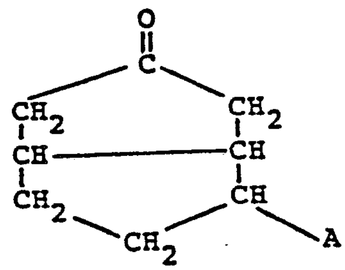

besitzt.

5. Verfahren nach Anspruch 4, worin A die Formel

$$—CH_2—CH_2—CH_2—C(R^4R^5)R^6$$

besitzt, in der
$R^4$ und $R^5$ unabhängig voneinander H, Methyl oder Ethyl sind,
$R^6$ COOH, CHO oder $C(R^7R^9)OR^8$ ist und
$R^7$, $R^8$ und $R^9$ unabhängig voneinander H, Methyl oder Ethyl sind, und die Verbindung eine cis-Brücke besitzt.

6. Verfahren nach Anspruch 4, worin $C(R^4R^5)R^6$
$—CH_2COOH$, $—CH_2CH_2OH$, $—CH_2CH(CH_3)OH$, $—CH_2CH(CH_2CH_3)OCH_3$, $—CH_2CHO$,
$—CH(CH_3)CH(CH_2CH_3)OH$ oder $—CH(CH_3)CH(CH_2CH_3)OCH_3$ ist.

7. Verfahren nach Anspruch 1, worin die Verbindung 2-(5-Methoxyhept-1-yl)-bicyclo[3.3.0]octan-7-on ist.

8. Verfahren nach Anspruch 1, worin die Verbindung 2-(5-Hydroxyhept-1-yl)-bicyclo[3.3.0]octan-7-on ist.

9. Verfahren nach Anspruch 1, worin die Verbindung 2-(5-Hydroxy-4-methylhept-1-yl)-bicyclo[3.3.0]octan-7-on ist.

10. Verfahren nach Anspruch 1, worin die Verbindung 2-(5-Methoxy-4-methylhept-1-yl)-bicyclo[3.3.0]octan-7-on ist.

**Revendications pour les Etats contractants: BE CH DE FR GB LI LU NL SE**

1. Composé de formule

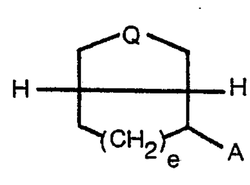

où
Q représente CO, CH—OR;
A représente $—CRR—(CR^1R^2)_nR_3$;
n est un nombre entier allant de un à huit;
R représente H ou un alcoyle inférieur (méthyle, éthyle, propyle, butyle);
$R^1$ et $R^2$ représentent indépendamment R, OR, COOH, CHO, ou $CR^1R^2$ représente CO;
$R_3$ représente H, COOR, OR, CHR—OR ou COR;
e est un nombre entier allant de 1 à 2;

sous réserve que le groupe A contienne au moins l'un des groupes COOR, CO, OR et COR, et pas plus qu'un des groupes COOH, CO ou COR, et où une liaison éthylénique peut être présente dans le noyau, ou un de ses sels pharmaceutiquement acceptables.

2. Composé de la revendication 1, où Q représente CO, CH—OH, CH—$OCH_3$ ou CH—$OCH_2CH_3$; e vaut 1; et n vaut de 1 à 4.

3. Composé de la revendication 1, où ledit composé est de formule

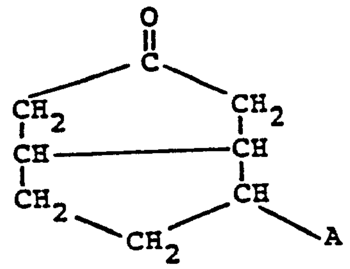

4. Composé de la revendication 3, où A est de formule

$$—CH_2—CH_2—CH_2—C(R^4R^5)R^6$$

ou

$R^4$ et $R^5$ représentent indépendamment H, un méthyle ou un éthyle;

$R^6$ représente COOH, CHO ou $C(R^7R^9)OR^8$; et

$R^7$, $R^8$ et $R^9$ représentent indépendamment H, un méthyle ou un éthyle, et le composé contient un pont cis.

5. Composé de la revendication 4, où $—C(R^4R^5)R^6$ représente

$$—CH_2COOH, —CH_2CH_2OH, —CH_2CH(CH_3)OH, —CH_2CH(CH_2CH_3)OCH_3,$$

$$—CH_2CHO, —CH(CH_3)CH(CH_2CH_3)OH, \text{ ou } —CH(CH_3)CH(CH_2CH_3)OCH_3.$$

6. Composé de la revendication 1 qui est la 2-(5-méthoxyhept-1-yl)-bicyclo-[3.3.0]octan-7-one.

7. Composé de la revendication 1 qui est la 2-(5-hydroxyhept-1-yl)-bicyclo-[3.3.0]octan-7-one.

8. Composé de la revendication 1 qui est la 2-(5-hydroxy-4-méthylhept-1-yl)-bicyclo-[3.3.0]octan-7-one.

9. Composé de la revendication 1 qui est la 2-(5-méthoxy-4-méthylhept-1-yl)-bicyclo-[3.3.0]octan-7-one.

10. Composition comprenant une quantité pharmaceutiquement efficace d'un composé de l'une quelconque des revendications 1—9.

11. Composition de la revendication 10 comprenant un support inerte approprié à l'administration locale dudit composé audit malade.

12. Composition de la revendication 11, où ledit support pharmaceutiquement inerte comprend un alcool, un baume, une suspension, une émulsion, un onguent, une crème, une poudre ou un liquide pulvérisé.

13. Composition de la revendication 11, où ledit support pharmaceutiquement inerte comprend un alcool.

14. Composition de l'une quelconque des revendications 10 à 13, qui est contenue dans un milieu à libération prolongée, ce qui fait que lorsqu'on applique ledit milieu à libération prolongée la composition est libérée sur la peau pendant une durée prolongée.

15. Composition de l'une quelconque des revendications 10 à 13 sous la forme d'un suppositoire pour l'administration rectale.

16. Composition de l'une quelconque des revendications 10 à 13 qui convient pour l'administration locale y compris un agent formant écran anti-ultraviolet.

17. Shampooing qui comprend la composition de l'une quelconque des revendications 10 à 13.

18. Composé de formule

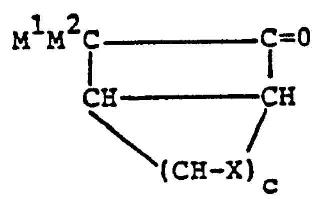

(II)

où

chaque X représente indépendamment H ou A;

chaque M représente indépendamment H, Cl, Br ou I;
A représente —CRR—$(CR^1R^2)_nR_3$;
n est un nombre entier allant de un à huit;
R représente H ou un alcoyle inférieur;
$R^1$ et $R^2$ représentent indépendamment R, OR, COOH, CHO, ou $CR^1R^2$ représente CO;
$R_3$ représente H, COOR, OR, CHR—OR ou COR;
c vaut trois ou quatre;
sous réserve qu'au moins un groupe A soit présent et que chaque groupe A contienne au moins un des groupes COOR, CHO, CO, OR et COR, et pas plus d'un des groupes COOH, CO ou COR, et où jusqu'a deux liaisons éthyléniques peuvent être présentes dans le noyau, ou un de ses sels pharmaceutiquement acceptables.

19. Composé de la revendication 18, où c vaut trois, constituant ainsi une structure cyclique bicyclo[3.2.0]heptan-7-one.

20. Composé de la revendication 19, qui contient un substituant X qui est un position 2 ou 4 du noyau alcoyl-bicyclo[3.2.0]heptan-7-one.

21. Composé de la revendication 20 où A représente

$$—CH_2—CH_2—CH_2—C(R^4R^5)R^6$$

où
$R^4$ et $R^5$ représentent indépendamment H, un méthyle ou un éthyle,
$R^6$ représente COOH, CHO ou $C(R^7R^9)OR^8$; et
$R^7$, $R^8$ et $R^9$ représentent indépendamment H, un méthyle ou un éthyle et le composé contient un pont cis.

22. Composé de la revendication 21, où —$C(R^4R^5)R^6$ représente

$$—CH_2COOH, —CH_2CH_2OH, —CH_2CH(CH_3)OH, —CH_2CH(CH_2CH_3)OCH_3,$$

$$—CH_2CHO, —CH(CH_3)CH(CH_2CH_3)OH, \text{ ou } —CH(CH_3)CH(CH_2CH_3)OCH_3.$$

23. Composé de la revendication 18 qui est la 6,6-dichloro-2-(5-méthoxyhept-1-yl)-bicyclo[3.2.0]-heptan-7-one.

**Revendications pour l'Etat contractant: AT**

1. Procédé de préparation d'un composé de formule

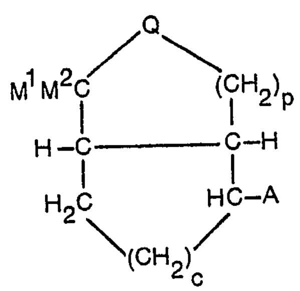

(I)

où
$M^1$ et $M^2$ représentent un hydrogène;
Q représente CO, CH—OR, ou —CR(OH);
A représente —CRR—$(CR^1R^2)_nR^3$;
n est un nombre entier allant de un à huit;
R représente H ou un alcoyle inférieur (méthyle, éthyle, propyle, butyle);
$R^1$ et $R^2$ représentent indépendamment R, OR, COOH, CHO, ou $CR^1R^2$ représente CO;
$R_3$ représente H, COOR, OR, CHR—OR ou COR;
e est un nombre entier allant de 1 à 2;
p est zéro ou un,
sous réserve que le groupe A contienne au moins l'un des groupes COOR, CHO, CO, OR et COR, et pas plus d'un groupe COOH, CO ou COR, et où une liaison éthylénique peut être présente dans le cycle, ou un de ses sels pharmaceutiquement acceptables, dans lequel on déshalogène un dihalogénure de formule I où $M^1$ et $M^2$ représentent un halogène, tous les autres symboles étant définis comme ci-dessus.

2. Procédé de la revendication 1 où l'on utilise le zinc comme catalyseur de déshalogénation dans un milieu acide.

3. Procédé pour préparer des composés des revendications 1 ou 2 et de formule I, où Q représente CO, CO—OH, CH—$OCH_3$ ou CH—$OCH_2CH_3$; c vaut 1; et n vaut de 1 à 4.

4. Procédé de la revendication 1, où ledit composé est de formule

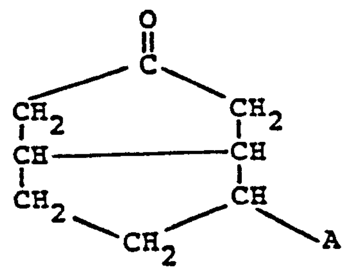

5. Procédé de la revendication 4, où A est de formule

$$—CH_2—CH_2—CH_2—C(R^4R^5)R^6$$

où

$R^4$ et $R^5$ représentent indépendamment H, un méthyle ou un éthyle;

$R^6$ représente COOH, CHO ou $C(R^7R^9)OR^8$; et

$R^7$, $R^8$ et $R^9$ représentent indépendamment H, un méthyle ou un éthyle, et le composé contient un pont cis.

6. Procédé de la revendication 5, où —$C(R^4R^5)R^6$ représente

$$—CH_2COOH, —CH_2CH_2OH, —CH_2CH(CH_3)OH, —CH_2CH(CH_2CH_3)OCH_3,$$

$$—CH_2CHO, —CH(CH_3)CH(CH_2CH_3)OH, \text{ ou } —CH(CH_3)CH(CH_2CH_3)OCH_3.$$

7. Procédé de la revendication 1 où le composé est la 2-(5-méthoxyhept-1-yl)-bicyclo[3.3.0]octan-7-one.

8. Procédé de la revendication 1 où le composé est la 2-(5-hydroxyhept-1-yl)-bicyclo[3.3.0]octan-7-one.

9. Procédé de la revendication 1 où le composé est la 2-(5-hydroxy-4-méthylhept-1-yl)-bicyclo[3.3.0]octan-7-one.

10. Procédé de la revendication 1 où le composé est la 2-(5-méthoxy-4-méthyl(hept-1-yl)-bicyclo[3.3.0]octan-7-one.